# EUROPÄISCHE PATENTSCHRIFT

(11) **EP 3 740 291 B1**
(45) Veröffentlichungstag und Bekanntmachung des Hinweises auf die Patenterteilung: **18.08.2021**
(21) Anmeldenummer: 19702803.8
(22) Anmeldetag: 11.01.2019
(51) Int. Cl.: A63B 69/00, A63B 102/02, A63B 63/00, A63B 69/38, A61B 1/00, A61B 1/227, A61B 5/01, A61B 5/00, A61N 5/06, A63B 71/02

(54) **TENNISWAND**
TENNIS WALL
MUR DE TENNIS

(30) Priorität: 18.01.2018 DE 102018000367
(43) Veröffentlichungstag der Anmeldung: 25.11.2020
(73) Patentinhaber: LENFERS, Alexander, 48691 Vreden (DE)
(72) Erfinder: LENFERS, Alexander, 48691 Vreden (DE)
(74) Vertreter: Herzog IP Patentanwalts GmbH
(86) Internationale Anmeldenummer: PCT/EP2019/025013
(87) Internationale Veröffentlichungsnummer: WO 2019/141509

(56) Entgegenhaltungen:
- EP-A2- 0 220 111
- US-B2- 9 155 952

## Beschreibung

Die vorliegende Erfindung betrifft eine Tenniswand und die Verwendung einer Tenniswand als Hilfsmittel im Tennistraining.

Tennis ist ein technisch anspruchsvoller Sport. Beim Tennis - besonders bei Anfängern - ist es daher sehr wichtig, die technischen Grundlagen zu erlernen, damit möglichst schnell ein Spiel mit Partnern zustande kommt, bei dem Ballwechsel entstehen.

Den Trainingsprozess unterstützt eine flexible Tenniswand. Mithilfe der Wand können Spieler in kurzer Zeit viele Wiederholungen durchführen, um Schläge zu trainieren. Durch regelmäßige Wiederholungen werden Spieler sicherer und erlernen in kurzer Zeit beispielsweise das richtige Timing, den perfekten Treffpunkt und guten Schwung. Aus dem Stand der Technik bekannte Tenniswände umfassen ein Tuch, welches schräg in einen Rahmen aufgespannt ist. Nachdem der Spieler den Schlag ausgeführt hat, trifft der Ball auf das Tuch. Beim Aufprall kann das Tuch - je nachdem, wie stark das Tuch im Rahmen aufgespannt ist - einen Teil des Impulses des Tennisballes aufnehmen, sodann rollt oder rutscht der Tennisball im gleichbleibenden Winkel entlang der Oberfläche des Tuches hinauf. Am Ende des Tuchs ist eine Sperre angebracht, die den Ball stoppt, der sodann wieder hinunterrollt oder rutscht. Unten am Tuch ist üblicherweise eine Abprallvorrichtung vorgesehen, mittels derer der Ball dann wieder in Richtung des Spielers befördert wird. Derartige Tenniswände werden beispielsweise von der Firma Tri-tennis® Nederland, s'Hertogenbosch, Niederlande, kommerziell vertrieben.

Der Nachteil dieser aus dem Stand der Technik bekannten Tenniswände besteht allerdings darin, dass die Zeitintervalle zwischen den Schlägen je nach Hersteller zwar etwas unterschiedlich, aber häufig nicht ausreichend lang genug sind. Bereits nach kurzer Zeit ist ein Spieler häufig nicht mehr in der Lage, den Arm schnell genug zurückzuführen, um den nächsten Schlag korrekt im Sinne der zu erlernenden, guten Tennistechnik auszuführen. In dem Tempo, in dem herkömmliche Tenniswände den Ball zurückspielen, ist ein gezieltes Training - besonders bei Anfängern und wenn diese alleine an der Wand spielen - nicht realistisch. Dies liegt darin begründet, dass der Winkel zwischen dem Tuch und dem Boden in herkömmlichen Tenniswänden entlang der gesamten Laufstrecke des Balles überall konstant ist und die Dimensionierung dieser Tennistrainingswände sowohl in der Tiefe, als auch in der Höhe begrenzt ist. Die Tuchoberfläche, auf welcher der Ball hinaufrollt oder hinaufrutscht, gestoppt wird und im Anschluss hinabrollt oder hinabrutscht, kann aber nicht beliebig lang ausgeführt werden, um die Zeitintervalle zu verlängern. Tennistrainingswände werden nämlich - auch im privaten Umfeld - ortsunabhängig betrieben und müssen daher auch üblichen Platzverhältnissen in Garagen, Carports, Gärten, Terrassen oder Kellerräumen Rechnung tragen.

In der Praxis kann man das Problem der zu kurzen Zeit zwischen den Ballwechseln teilweise dadurch umgehen, dass man den Ball nach dem ersten Aufprall ein weiteres Mal auf dem Boden aufprallen lässt und dann erst den nächsten Schlag durchführt. Dies setzt aber voraus, dass der Boden, auf dem die Tennistrainingswand steht, sehr eben ist (beispielsweise ein ebener Betonboden), so dass der Ball nicht verspringen kann, und dass zudem ausreichend Platz nach hinten für den zweiten Aufprall zur Verfügung steht. Ferner ist diese Lösung nur für geübte Spieler realisierbar. Je weiter ein Spieler von der Wand entfernt steht, desto genauer muss er zielen, um die Fläche der Tenniswand zu treffen. Besonders Anfänger sollten daher so nah wie möglich an der Wand stehen, um diese auch optimal zu treffen. Dokument EP-A-0220111 beschreibt ein Trainingsgerät für Tennis mit einem von einem Rahmen getragenen Netz.

Der vorliegenden Erfindung lag die Aufgabe zugrunde, die aus dem Stand der Technik bekannten Nachteile im Zusammenhang mit Tenniswänden zu überwinden.
Insbesondere lag der vorliegenden Erfindung die Aufgabe zugrunde, eine Tenniswand anzugeben, bei der das Zeitintervall zwischen zwei Schlägen verlängert und darüber hinaus auch variabel eingestellt werden kann, ohne dabei die Dimensionierung des Tuches entscheidend ändern zu müssen. Die Tenniswand sollte sich weiterhin vorteilhafterweise dadurch auszeichnen, dass sie nicht nur kostengünstig herstellbar ist, sondern möglichst auch auf unebenen Untergründen und bei möglichst geringem Platzbedarf ein gezieltes Tennistraining auch für Anfänger ermöglicht.

Gelöst wird diese Aufgabe durch eine Tenniswand wie in Anspruch 1 beansprucht.

Überraschenderweise hat sich gezeigt, dass sich die Rücklaufgeschwindigkeit eines Tennisballes bei einer Tenniswand in einfacher Weise, zuverlässig und vor allem sehr variabel einstellen lässt, in dem man am oberen Ende des Tuches eine Vertiefung vorsieht, in die der Ball nach dem Hinaufrollen oder Hinaufrutschten am Tuch eindringen und in der er für eine bestimmte, variierbare Verweilzeit verbleiben kann. Nachdem der Spieler den Schlag ausgeführt hat, trifft der Ball auf die Tuchoberfläche. Beim Aufprall kann das Tuch - je nachdem, wie stark das Tuch im Rahmen aufgespannt ist - einen Teil des Impulses des Tennisballes aufnehmen. Sodann rollt oder rutscht der Tennisball im gleichbleibenden Winkel entlang der Oberfläche des Tuches nach oben. Am oberen Ende des Tuchs dringt der Ball in die Vertiefung ein und wird dort gestoppt. Im Anschluss daran rollt oder rutscht der Ball wieder aus der Vertiefung heraus und sodann an der Tuchoberfläche wieder nach unten. Unten angekommen trifft der Ball beispielsweise auf ein Brett oder kleines Trampolin, das am oder im Gestell verankert ist oder auf dem Boden steht, oder der Ball trifft direkt auf den Boden. Der Spieler steht vor der Tenniswand und führt nach dem Aufprall den nächsten Schlag aus. Dieser Vorgang wiederholt sich nun immer und immer wieder im gleichen Rhythmus und Zeitintervall.
Die erfindungsgemäße Tenniswand umfasst als einen Vorrichtungsbestandteil einen auf dem Boden aufstellbaren Rahmen. Der Rahmen der erfindungsgemäßen Tenniswand kann aus jedem Material gefertigt sein, welches der Fachmann als geeignet für die Konstruktion eines Rahmens einer Tenniswand ansehen würde. Vorzugsweise ist der Rahmen aus Metall, beispielsweise aus Aluminium, aus verzinktem Stahl oder aus Edelstahl oder aber aus einer Kombination dieser Materialien, aus Kunststoff, beispielsweise Polyethylen, Polypropylen, PET oder Polycarbonat oder aus Holz gefertigt, wobei die Verwendung eines Aluminiumrahmens oder eines Rahmens aus dünnwandigem, verzinktem Stahl oder Edelstahl im Hinblick auf die Leichtigkeit, die Stabilität und die Korrosionsbeständigkeit besonders bevorzugt ist. Der Rahmen kann weiterhin Räder aufweisen, um die Tenniswand einfacher auf dem Boden von einem Ort zum anderen bewegen zu können.

Die erfindungsgemäße Tenniswand umfasst als einen weiteren Vorrichtungsbestandteil ein in diesem Rahmen aufgespanntes Tuch, wobei dieses Tuch vorzugsweise eine von einer oberen Kante, einer unteren Kante, einer linken Seitenkante und einer rechten Seitenkante begrenzte Tuchoberfläche aufweist. Das Tuch ist dabei derart im Rahmen aufgespannt, dass eine im Wesentlichen ebene Tuchoberfläche gebildet wird und dass ein auf die Tuchoberfläche auftreffender Tennisball an der Tuchoberfläche hinaufrollen und anschließend wieder hinunterrollen kann.

Das Tuch der erfindungsgemäßen Tenniswand kann ebenfalls aus jedem Material gefertigt sein, welches der Fachmann als geeignet für ein solches Tuch ansehen würde. Vorzugsweise ist das Tuch aus Kunststoff, beispielsweise aus einer LKW-Plane, einem luftdurchlässigen, netzartigen Werbebanner ("Meshbanner") beispielsweise aus PVC, aus Schaumstoff oder aus textilähnlichen Materialen gefertigt. Besonders bevorzugt sind witterungsbeständige Materialien, so dass die Tenniswand auch im Freien betrieben bzw. genutzt werden kann.

Weiterhin ist es erfindungsgemäß bevorzugt, dass das Tuch eine Breite in einem Bereich von 50 bis 300 cm, bevorzugt im Bereich von 80 bis 250 cm und noch mehr bevorzugt in einem Bereich von 100 bis 180 cm und eine Länge (gemessen bis zum Beginn der Faltung, sofern die Vertiefung - wie nachfolgend noch erörtert - über eine Faltung des Tuches realisiert wird) in einem Bereich von 100 bis 400 cm, bevorzugt im Bereich von 150 bis 380 cm und noch mehr bevorzugt in einem Bereich von 180 bis 330 cm aufweist. Die Dicke des Tuches liegt vorzugsweise in einem Bereich von 0,1 bis 30 mm, besonders bevorzugt in einem Bereich von 0,5 bis 6 mm. Sofern die Vertiefung - wie nachfolgend noch erörtert - über eine Faltung des Tuches realisiert wird, sollte die Dicke des Tuches so bemessen sein, dass es sich problemlos falten lässt.

Das Aufspannen des Tuches im Rahmen kann über Zugfedern, Gummibänder, Expander-Bänder oder andere, vorzugsweise dehnbare Befestigungs-Hilfsmittel erfolgen. Dabei ist sicherzustellen, dass durch das Aufspannen des Tuches im Rahmen eine im wesentlichen ebene Tuchoberfläche gebildet wird, auf der ein Tennisball, der mit einer bestimmten Mindestgeschwindigkeit auf das Tuch prallt, auf der Oberfläche des Tuches hinaufrollen und anschließend auch hinabrollen kann. Durch das Aufspannen des Tuches im Rahmen mittels dehnbarer Befestigungsvorrichtungen wird sichergestellt, dass das Tuch sehr anwenderfreundlich aufgespannt werden kann, sogleich eine ebene Tuchoberfläche entsteht und das Tuch einen Teil des Impulses des auftreffenden Tennisballes aufnehmen kann.

Erfindungsgemäß ist es weiterhin bevorzugt, dass das Tuch derart aufgespannt ist, dass zwischen der ebenen Tuchoberfläche und dem Boden ein Winkel α im Bereich von 25 bis 70 Grad, besonders bevorzugt in einem Bereich von 35 bis 55 Grad eingeschlossen wird. Das Tuch ist demnach derart im Rahmen aufgespannt (bzw. der Rahmen ist derart konstruiert), dass die durch das Tuch gebildete ebene Tuchoberfläche schräg in Richtung des Spielers zum Boden geneigt ist. Durch diese Schrägstellung des Tuches wird sichergestellt, dass ein auf die Oberfläche des Tuches aufprallender Ball für eine bestimmte Wegstrecke auf der Tuchoberfläche nach oben und anschließend wieder nach unten rollen oder rutschen kann.

Die erfindungsgemäße Tenniswand ist nun dadurch gekennzeichnet, dass am oberen Ende des Tuches eine Vertiefung vorgesehen ist, wobei die Vertiefung derart ausgestaltet und angeordnet ist, dass ein Tennisball, wenn er an der Tuchoberfläche hinaufgerollt oder hinaufgerutscht ist, anschließend in die Vertiefung eindringt, danach aus der Vertiefung wieder hinausrollt oder hinausrutscht und erst dann an der Tuchoberfläche hinunterrollt oder hinunterrutscht. Vorzugsweise wird dabei unter einer "Vertiefung" eine Konstruktion verstanden, die am oberen Ende der Tuchoberfläche vorgesehen ist (bzw., wie nachfolgend im Zusammenhang mit einer besonders bevorzugten Ausgestaltung der erfindungsgemäßen Tenniswand genauer erörtert, die durch Faltung im oberen Bereich des Tuches gebildet wird) und die eine sich vorzugsweise unmittelbar an das obere Ende der ebenen Tuchoberfläche angrenzende ebene oder im Radius leicht gewölbte, erste Oberfläche umfasst. Diese erste Oberfläche schließt einen im Vergleich zum vorstehend beschriebenen Winkel α verminderten Winkel β zwischen sich und dem Boden ein. Vorzugsweise rollt oder rutscht ein in die Vertiefung eingedrungener Tennisball letztlich entlang dieser im Vergleich zur Tuchoberfläche (i. e. im Vergleich zu derjenigen Fläche des Tuches, auf die der Tennisball zunächst auftrifft) weniger stark geneigten ersten ebenen Oberfläche (die der nachfolgend genauer beschriebenen ersten Tuchteiloberfläche entspricht) wieder aus der Vertiefung hinaus.

Gemäß einer besonders bevorzugten Ausgestaltung der erfindungsgemäßen Tenniswand wird die Vertiefung durch eine Falte im oberen Bereich des Tuches gebildet. Vorzugsweise weist diese Falte die Form eines Grabens auf, der im Wesentlichen in horizontaler Richtung von der linken zur rechten Seite des Tuches verläuft und sich vorzugsweise über die gesamte Breite des Tuches erstreckt.

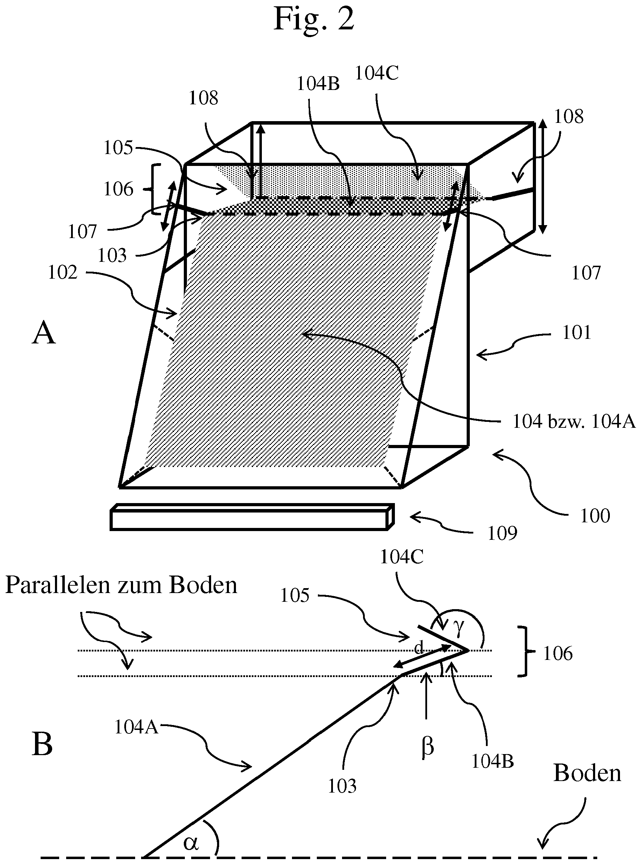

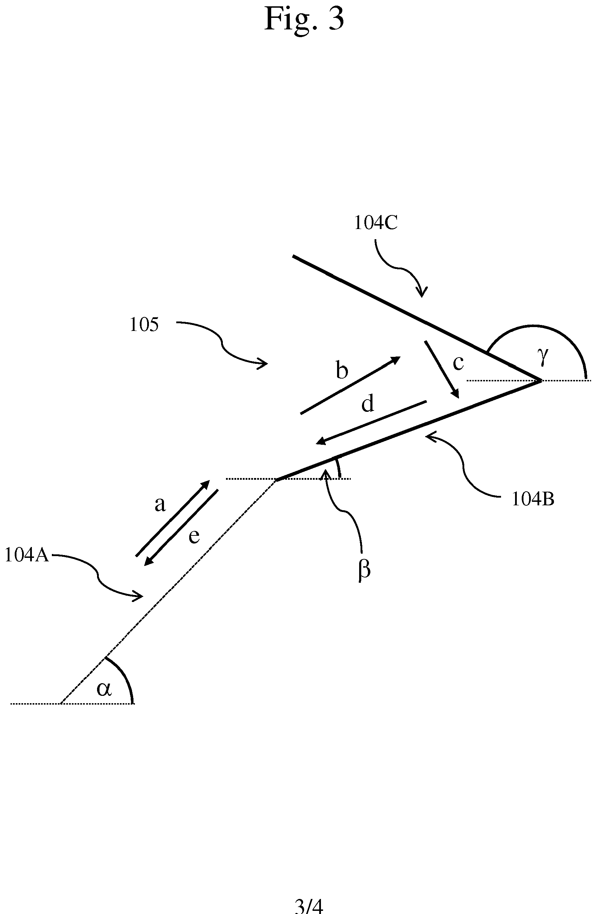

Besonders bevorzugt ist es in diesem Zusammenhang, dass diese Vertiefung bzw. diese Falte durch mindestens zwei Faltungen des Tuches gebildet werden, wie es durch die beiden gestrichelten Linien in der Figur 2A beispielhaft veranschaulicht ist. Die Faltungen verlaufen jeweils in horizontaler Richtung von der linken zur rechten Seite des Tuches und erstrecken sich über die gesamte Breite des Tuches. Dabei ist es weiterhin besonders bevorzugt, dass durch eine erste und eine zweite Faltung
- eine ebene Tuchhauptoberfläche,
- eine an die ebene Tuchhauptoberfläche angrenzende erste ebene Tuchteiloberfläche und
- eine an die erste ebene Tuchteiloberfläche angrenzende zweite ebene Tuchteiloberfläche gebildet werden. Die Tuchhauptoberfläche entspricht dabei in ihrer Lage und Orientierung der vorstehend beschriebenen Tuchoberfläche, i. e. derjenigen Oberfläche des Tuches, auf die der Tennisball bei ordnungsgemäßer Nutzung der erfindungsgemäßen Tenniswand zunächst auftrifft (das obere Ende des Tuches der erfindungsgemäßen Tenniswand, an der die Vertiefung vorgesehen ist, entspricht in diesem Fall dem oberen Ende der Tuchhauptoberfläche). Dabei ist es besonders vorteilhaft, wenn die erste und die zweite ebene Tuchteiloberfläche so geneigt sind, dass ein Ball, der die Tuchhauptoberfläche hinaufgerollt oder hinaufgerutscht und von dort in die Falte eingedrungen ist, vorteilhafterweise zunächst auf die zweite ebene Tuchteiloberfläche prallt und, durch die Schrägstellung dieser ebenen Tuchteiloberfläche, in Richtung der ersten ebenen Tuchteiloberfläche geleitet wird, von der aus er dann aus der Falte hinausrollt oder hinausrutscht (siehe hierzu auch die Figur 3). In diesem Zusammenhang ist es besonders bevorzugt, dass
- zwischen der ebenen Tuchhauptoberfläche und dem Boden ein Winkel α eingeschlossen wird;
- zwischen der ersten ebenen Tuchteiloberfläche und dem Boden (bzw. zwischen der ersten ebenen Tuchteiloberfläche und einer parallel zum Boden angeordneten Ebene) ein Winkel β eingeschlossen wird, der gegenüber dem Winkel α um mindestens 5 Grad, bevorzugt um mindestens 15 Grad und besonders bevorzugt um mindestens 25 Grad reduziert ist;
- zwischen der zweiten ebenen Tuchteiloberfläche und dem Boden (bzw. zwischen der zweiten ebenen Tuchteiloberfläche und einer parallel zum Boden angeordneten Ebene) ein Winkel γ eingeschlossen wird, der gegenüber dem Winkel α um mindestens 75 Grad, bevorzugt um mindestens 100 Grad und besonders bevorzugt im mindestens 120 Grad erhöht ist.

Vorzugsweise liegt der Winkel α in einem Bereich von 25 bis 70 Grad, besonders bevorzugt in einem Bereich von 35 bis 55 Grad, der Winkel β in einem Bereich von 5 bis 30 Grad, besonders bevorzugt in einem Bereich von 8 bis 20 Grad und der Winkel γ in einem Bereich von 130 bis 190 Grad, besonders bevorzugt in einem Bereich 150 bis 180 Grad.

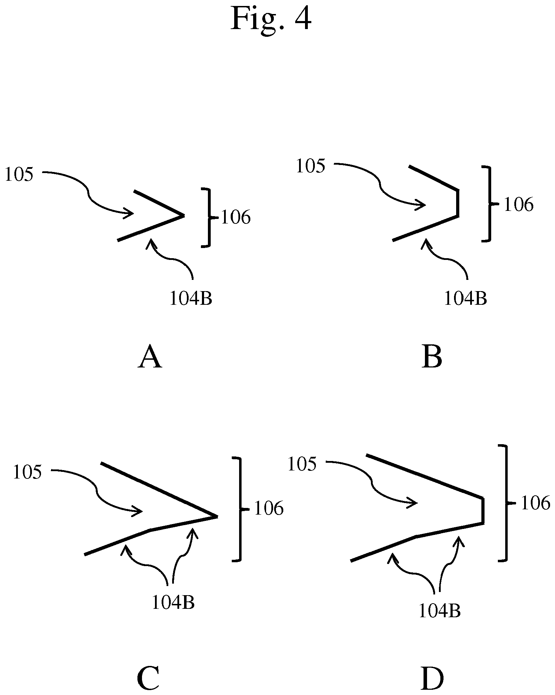

Die Art und die Anzahl der Faltungen des Tuches zur Bildung der Vertiefung bzw. der Falte kann beliebig variiert werden. Im einfachsten und vorstehend beschriebenen Fall von lediglich zwei Faltungen wird eine spitz zulaufende, im Profil keilförmige, Falte gebildet. Denkbar sind aber auch drei oder mehr Faltungen, so dass sich auch komplexere Faltenprofile herstellen lassen (siehe hierzu auch die Figur 4). Denkbar ist insbesondere auch, den Winkel β schrittweise über zwei oder mehr aneinandergrenzende Tuchteiloberflächen zu reduzieren, wie es beispielsweise in den Figuren 4C und 4D gezeigt ist.

Im Zusammenhang mit dieser besonders bevorzugten Ausführungsform der erfindungsgemäßen Tenniswand, in der die Vertiefung über eine Falte im oberen Bereich des Tuches gebildet wird, ist es weiterhin bevorzugt, dass das Tuch an der linken und der rechten Seite des oberen Endes des Tuches jeweils über eine erste Faltungs-Fixierung und eine zweite Faltungs-Fixierung mit dem Rahmen verbunden ist, und wobei mittels dieser Faltungs-Fixierungen die erste und die zweite Faltung des Tuches unter Bildung einer Falte erfolgt. Sofern die Falte über mehr als zwei Faltungen gebildet wird, sind entsprechend mehr Faltungs-Fixierungen vorzusehen. Als Faltungs-Fixierung können alle Materialien bzw. Vorrichtungen gewählt werden, die der Fachmann als geeignet empfindet. Dies können beispielsweise Stangen zwischen den Rahmen-Enden oder Seile aus beispielsweise PVC, Nylon oder Stahl sein oder aber auch Fixierungs-Hilfsmittel, wie beispielsweise Gummi- und Expander-Schlingen aus Kautschuk oder Zugfedern aus Metall. In diesem Zusammenhang hat es sich weiterhin als vorteilhaft erwiesen, dass die Form der Falte durch eine am Rahmen angebrachte und in ihrer Position variable erste und/oder variable zweite Faltungs-Fixierung einstellbar ist. Auf diese Art und Weise können vor allem die Winkel β und γ und auch die Länge d (siehe Fig. 2B), entlang derer der Tennisball auf der durch die erste Faltung gebildeten ersten ebenen Tuchteiloberfläche in der Falte entlangläuft oder entlangrutscht, und damit letztlich die Verweilzeit des Tennisballes in der Falte variiert werden.

Vorzugsweise liegt die Verweilzeit (i. e. die Zeit, die zwischen dem Eindringen des Balles in die Vertiefung und dem Verlassen der Vertiefung vergeht) eines Tennisballes in der Vertiefung, besonders bevorzugt in der durch die mindestens zwei Faltungen des Tuches gebildeten Falte, bei mindestens 0,1 Sekunden, besonders bevorzugt bei mindestens 0,5 Sekunden. Insbesondere kann sie in einem Bereich von 0,1 bis 3 Sekunden, bevorzugt in einem Bereich von 0,25 bis 2 Sekunden und noch mehr bevorzugt in einem Bereich von 0,5 bis 1 Sekunde liegen.

Die erfindungsgemäße Tenniswand kann weiterhin eine Abprall-Vorrichtung umfassen, welche derart angeordnet ist, dass ein Tennisball, nachdem er an der Tuchoberfläche hinuntergerollt oder hinuntergerutscht ist, auf diese Abprall-Vorrichtung fällt und von ihr in Richtung des Spielers befördert wird. Eine solche Abprall-Vorrichtung kann mit dem Rahmen verbunden bzw. Teil des Rahmens sein oder als separater Bestandteil der Tenniswand auf dem Boden vor dem Rahmen platziert werden. Beispiele geeigneter Abprall-Vorrichtungen sind einfache, am Rahmen fixierte Leisten oder Bretter, oder ein vor der Tenniswand lokalisiertes Trampolin oder Brett mit starren oder variablem Winkel. Bei Änderungen in der Ausrichtung derjenigen Oberfläche der Abprall-Vorrichtungen, auf die der Ball nach dem Verlassen der Tuchoberfläche prallt, kann der Winkel variiert werden, in dem der Ball in Richtung des Spielers befördert wird.

Die vorliegende Erfindung betrifft auch die Verwendung der erfindungsgemäßen Tenniswand als Hilfsmittel im Tennistraining, insbesondere im Tennistraining im Profibereich.

Die Erfindung wird nun anhand von nicht limitierenden Figuren näher erläutert.
Figur 1A zeigt eine aus dem Stand der Technik bekannte Tenniswand 100 in einer Ansicht von vorne, während Figur 1B die Form des Tuches 102 in einer Seitenansicht zeigt.
Figur 2A zeigt eine erfindungsgemäße Tenniswand 100 in einer Ansicht von vorne, während Figur 2B die Form des Tuches 102 in einer Seitenansicht zeigt.
Figur 3 zeigt den Verlauf des Balles beim Eindringen in die Vertiefung bzw. Falte 105.
Figuren 4A bis 4D zeigen verschiedene Profile einer durch Faltung des oberen Endes 103 des Tuches 102 gebildeten Falte 105.

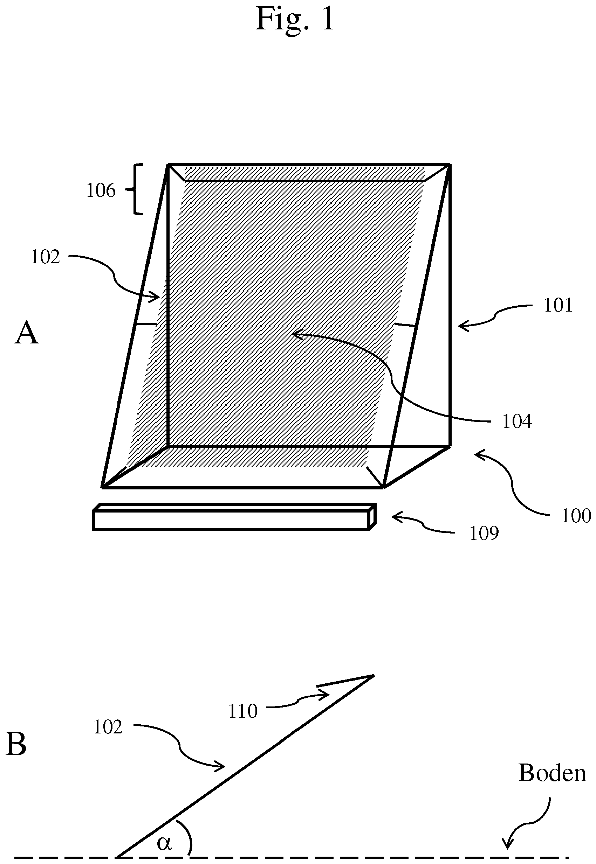

Figur 1A zeigt eine aus dem Stand der Technik bekannte Tenniswand 100 in einer Ansicht von vorne, während Figur 1B die Form des Tuches in einer Seitenansicht zeigt. Eine solche herkömmliche Tenniswand 100, wie sie beispielsweise von der Firma Tri-tennis® Nederland, s'Hertogenbosch, Niederlande kommerziell vertrieben wird, umfasst einen auf dem Boden aufgestellten Rahmen 101 und ein in diesem Rahmen aufgespanntes Tuch 102, welches derart im Rahmen aufgespannt ist, dass es eine Schräg zum Boden in Richtung des Spielers verlaufende, im wesentlichen ebene Tuchoberfläche 104 bildet. Zwischen der Tuchoberfläche 104 und dem Boden wird der Winkel α eingeschlossen, der üblicherweise in einem Bereich von 35 bis 55 Grad liegt. Trifft ein Ball (in der Figur 1A nicht gezeigt) auf die ebene Tuchoberfläche 104, so kann das Tuch 102 beim Aufprall des Tennisballs - je nachdem, wie stark das Tuch 102 im Rahmen 101 eingespannt ist - einen Teil des Impulses des Tennisballes aufnehmen. Dieser läuft im Anschluss im gleichbleibenden Winkel an der Tuchoberfläche 104 hinauf, bis er - sofern er mit ausreichender Geschwindigkeit auf die Tenniswand 100 bzw. die Tuchoberfläche 104 gespielt wird - auf den Endpunkt 110 im oberen Bereich 106 der Tuchoberfläche trifft, der bei einer herkömmlichen Tenniswand durch eine einfache Faltung des Tuches 102 am oberen Ende des Tuches 102 gebildet wird. Dort wird der Ball gestoppt und läuft sodann an der Tuchoberfläche 104 wieder hinunter. Am unteren Ende des Tuches 102 angekommen, trifft der Ball auf eine Abprall-Vorrichtung 109, bei der es sich beispielsweise um eine am Rahmen 101 fixierte Leiste oder ein vor der Tenniswand 100 positioniertes Winkelbrett oder Trampolin handelt, und springt von dort wieder in Richtung des Spielers, der dann den nächsten Schlag ausführen kann.

Figur 2A zeigt eine erfindungsgemäße Tenniswand 100 in einer Ansicht von vorne, während Figur 2B die Form des Tuches in einer Seitenansicht zeigt. In ihrem Grundaufbau ist diese mit herkömmlichen Tenniswänden vergleichbar. Sie umfasst ebenfalls einen auf dem Boden aufgestellten Rahmen 101 und ein in diesem Rahmen aufgespanntes Tuch 102, welches derart im Rahmen 101 aufgespannt ist, dass es eine Schräg zum Boden in Richtung des Spielers verlaufende, im wesentlichen ebene Tuchoberfläche 104 bildet. Im Gegensatz zu einer herkömmlichen Tenniswand ist jedoch bei der erfindungsgemäßen Tenniswand 100 am oberen Ende 103 des Tuches 102 eine Vertiefung 105 vorgesehen, in die ein auf die Tuchoberfläche 104 auftreffender Ball (auch in Figur 2A nicht gezeigt), nachdem er an der Tuchoberfläche 104 hinaufgerollt oder hinaufgerutscht ist, eindringt. In der einfachsten, in Figur 2A dargestellten Ausführungsform wird diese Vertiefung 105 durch eine Falte 105 im oberen Bereich 106 des Tuches 102 gebildet, welche durch zwei Faltungen realisiert wird (erkennbar an den gestrichelten Linien in der Figur 2A). Dabei werden durch die erste und die zweite Faltung eine ebene Tuchhauptoberfläche 104A (die in ihrer Lage und Orientierung der Tuchoberfläche 104 entspricht), eine sich an die Tuchhauptoberfläche 104A anschließende erste ebene Tuchteiloberfläche 104B und eine sich an die erste ebene Tuchteiloberfläche 104B anschließende zweite ebene Tuchteiloberfläche 104C gebildet. Das obere Ende 103 des Tuches 102, an dem die Vertiefung 105 vorgesehen ist, entspricht in diesem Fall dem oberen Ende der Tuchhauptoberfläche 104A. Dabei sind die erste und die zweite ebene Tuchteiloberfläche (104B, 104C) so geneigt, dass ein Tennisball, der die Tuchhauptoberfläche 104A hinaufgerollt oder hinaufgerutscht und von dort in die Falte 105 eingedrungen ist, vorteilhafterweise zunächst auf die zweite Tuchteiloberfläche 104C prallt und, durch die Stellung dieser Tuchteiloberfläche 104C, in Richtung der ersten Tuchteiloberfläche 104B geleitet wird, von der aus er dann aus der Falte 105 hinausrollt oder hinausrutscht. Diese erste ebene Tuchteiloberfläche 104B hat die Länge d (siehe Figur 2B). Im Vergleich zum Winkel α zwischen der Tuchhauptoberfläche 104A und dem Boden wird zwischen der ersten ebenen Tuchteiloberfläche 104B und dem Boden (bzw. zwischen der ersten ebenen Tuchteiloberfläche 104B und einer parallel zum Boden angeordneten Ebene) ein im Vergleich zum Winkel α reduzierter Winkel β eingeschlossen (die erste ebene Tuchteiloberfläche 104B ist also weniger stark geneigt als die Tuchhauptoberfläche 104A), während zwischen der zweiten ebenen Tuchteiloberfläche 104C und dem Boden (bzw. zwischen der zweiten ebenen Tuchteiloberfläche 104C und einer parallel zum Boden angeordneten Ebene) ein im Vergleich zum Winkel α erhöhter Winkel γ eingeschlossen wird. Wie der Figur 2A weiterhin entnommen werden kann, werden die beiden Faltungen durch entsprechende Faltungs-Fixierungen 107 und 108 bewerkstelligt, mit denen die linke und die rechte Seite des oberen Endes des Tuches oder mit denen das gesamte Tuch über seine Breite gemessen mit dem Rahmen verbunden werden bzw. wird (die linke obere Faltungsfixierung 108 ist in der Figur 2A nicht zu erkennen, da sie hinter dem Tuch 102 liegt). Bei diesen Faltungsfixierungen 107 und 108 können alle Materialien, die ein Fachmann als geeignet empfindet, herangezogen werden. Dies können beispielsweise Stangen zwischen den Rahmenenden oder Seile aus beispielsweise PVC, Nylon oder Stahl sein oder aber auch Fixierungs-Hilfsmittel, wie beispielsweise Gummi- und Expander-Schlingen aus Kautschuk oder Zugfedern aus Metall. Wie ebenfalls durch die Doppelpfeile in der Figur 2A veranschaulicht, kann die erste (untere) Faltungsfixierung 107, die zweite (obere) Faltungs-Fixierung 108 oder können beide Faltungs-Fixierungen 107 und 108 in der Höhe variabel einstellbar sein. Auf diese Art und Weise lassen sich das Profil der Falte und damit insbesondere die Winkel β und γ sowie auch die Länge d und so schließlich auch die Verweilzeit des Balles in der Falte 105 variieren. Trifft ein Ball auf die ebene Tuchhauptoberfläche 104A, so kann das Tuch 102 - je nachdem, wie stark das Tuch 102 im Rahmen 101 aufgespannt ist - einen Teil des Impulses des Tennisballes übernehmen. Dieser läuft an der Tuchhauptoberfläche 104A hinauf, bis er - sofern er mit ausreichender Geschwindigkeit auf die Tenniswand gespielt worden ist - in die Vertiefung bzw. Falte 105 eindringt. Hier kann er, wie es in der Figur 3 gezeigt ist, beispielsweise gegen die zweite Tuchteiloberfläche 104C prallen, von wo er, bedingt durch das Ausmaß der Schrägstellung dieser Tuchteiloberfläche, in Richtung der ersten Tuchteiloberfläche 104B geleitet wird. An dieser rollt oder rutscht der Ball aus der Falte 105 hinaus und gelangt wieder auf die Tuchhauptoberfläche 104A, an der er hinunterrollt oder hinunterrutscht. Am unteren Ende des Tuches 102 angekommen, kann der Ball wieder auf eine herkömmliche Abprall-Vorrichtung 109 (oder auch auf den Boden) treffen, von wo er wieder in Richtung des Spielers springt, der dann den nächsten Schlag ausführen kann.

Figur 3 zeigt beispielhaft einen möglichen Verlauf eines Tennisballes auf der erfindungsgemäßen Tenniswand 100. Der Ball rollt oder rutscht die Tuchhauptoberfläche 104A hinauf (Pfeil a). Oben angekommen, dringt er in die Falte 105 ein und springt - sofern der fest genug geschlagen worden ist - gegen die zweite Tuchteiloberfläche 104C (Pfeil b). Von dort wird er in Richtung der ersten Tuchteiloberfläche 104B befördert (Pfeil c) (oder gegebenenfalls auch in Richtung der Ecke, die im Kontaktbereich zwischen der ersten Tuchteiloberfläche 104B und der zweiten Tuchteiloberfläche 104C gebildet wird), auf der er dann aus der Falte 105 herausrollt oder herausrutscht (Pfeil d) und anschließend an der Tuchhauptoberfläche 104A hinunterrollt oder hinunterrutscht (Pfeil e). Denkbar (und in Figur 3 nicht gezeigt) ist aber auch, dass der Ball - wenn er weniger stark geschlagen worden ist - nach dem Eindringen in die Falte 105 auf der ersten ebenen Tuchteiloberfläche 104B hinaufrollt oder hinaufrutscht, bis er auf die zweite ebene Tuchteiloberfläche 104C trifft und dort gebremst wird. Anschließend läuft er dann wieder entlang der ersten ebenen Tuchteiloberfläche 104B aus der Falte 105 hinaus.

Figuren 4A bis 4D zeigen verschiedene Profile einer durch Faltung im oberen Bereich 106 des Tuches 102 gebildeten Falte 105. Die in Figur 4A gezeigte Falte 105 entspricht derjenigen in den Figuren 2A und 2B. Die in Figur 4B gezeigte Falte wird durch eine dreifache Faltung im oberen Bereich 106 des Tuches 102 gebildet. Figuren 4C und 4D zeigen Ausgestaltungen einer Falte 105, bei denen der Winkel α schrittweise über zwei aneinandergrenzende Tuchteiloberflächen 104B reduziert wird.

### BEZUGSZEICHENLISTE

- 100: Tenniswand
- 101: Rahmen
- 102: Tuch
- 103: oberes Ende des Tuches 102
- 104: ebene Tuchoberfläche
- 104A: ebene Tuchhauptoberfläche
- 104B: sich nach der ersten Faltung an die Tuchhauptoberfläche 104A anschließende erste ebene Tuchteiloberfläche
- 104C: sich nach der zweiten Faltung an die erste ebene Tuchteiloberfläche 104B anschließende zweite ebene Tuchteiloberfläche
- 105: Vertiefung (bzw. Falte)
- 106: oberer Bereich des Tuches 102
- 107: erste Faltungs-Fixierung
- 108: zweite Faltungs-Fixierung
- 109: Abprall-Vorrichtung
- 110: Endpunkt einer Tenniswand des Standes der Technik

## Patentansprüche

1. Tenniswand (100), umfassend
- einen auf dem Boden aufstellbaren Rahmen (101) und
- ein in diesem Rahmen (101) aufgespanntes Tuch (102),
wobei das Tuch (102) derart aufgespannt ist, dass eine ebene Tuchoberfläche (104) gebildet wird, dass zwischen der ebenen Tuchoberfläche (104) und dem Boden ein Winkel α im Bereich von 25 bis 70 Grad eingeschlossen wird und dass ein auf die Tuchoberfläche (104) auftreffender Tennisball an der Tuchoberfläche (104) hinaufrollt oder hinaufrutscht und anschließend wieder hinunterrollt oder hinunterrutscht,
wobei am oberen Ende (103) des Tuches (102) eine Vertiefung (105) vorgesehen ist, welche durch eine Falte (105) im oberen Bereich (106) des Tuches (102) gebildet wird und welche die Form eines Grabens aufweist, der in horizontaler Richtung von der linken zur rechten Seite des Tuches (102) verläuft und sich über die gesamte Breite des Tuches (102) erstreckt,
**dadurch gekennzeichnet, dass** die Vertiefung (105) eine unmittelbar an das obere Ende (103) der ebenen Tuchoberfläche (104) angrenzende erste Tuchteiloberfläche (104B) umfasst, welche einen im Vergleich zum Winkel α verminderten Winkel β zwischen sich und dem Boden einschließt,
wobei die Vertiefung (105) derart ausgestaltet und angeordnet ist, dass ein Tennisball, wenn er an der Tuchoberfläche (104) hinaufgerollt oder hinaufgerutscht ist, anschließend in die Vertiefung (105) eindringt, entlang dieser im Vergleich zur ebenen Tuchoberfläche (104) weniger stark geneigten ersten Tuchteiloberfläche (104B) aus der Vertiefung (105) wieder hinausrollt oder hinausrutscht und erst dann an der Tuchoberfläche (104) hinunterrollt oder hinunterrutscht.

2. Tenniswand (100) nach Anspruch 1, **dadurch gekennzeichnet, dass** die Falte (105) durch mindestens zwei Faltungen des Tuches (102) gebildet wird.

3. Tenniswand (100) nach Anspruch 2, **dadurch gekennzeichnet, dass** durch eine erste und eine zweite Faltung eine ebene Tuchhauptoberfläche (104A), die an die Tuchhauptoberfläche (104A) angrenzende erste ebene Tuchteiloberfläche (104B) und eine an die erste ebene Tuchteiloberfläche (104B) angrenzende zweite ebene Tuchteiloberfläche (104C) gebildet werden, wobei
- zwischen der ersten ebenen Tuchteiloberfläche (104B) und dem Boden ein Winkel β eingeschlossen wird, der gegenüber dem Winkel α um mindestens 5 Grad reduziert ist;
- zwischen der zweiten ebenen Tuchteiloberfläche (104C) und dem Boden ein Winkel γ eingeschlossen wird, der gegenüber dem Winkel α um mindestens 75 Grad erhöht ist.

4. Tenniswand nach Anspruch 3, **dadurch gekennzeichnet, dass** der Winkel β in einem Bereich von 5 bis 30 Grad und der Winkel γ in einem Bereich von 130 bis 190 Grad liegen.

5. Tenniswand (100) nach Anspruch 3 oder 4, **dadurch gekennzeichnet, dass** das Tuch (102) an der linken und der rechten Seite des oberen Endes des Tuches (102) jeweils über eine erste Faltungs-Fixierung (107) und eine zweite Faltungs-Fixierung (108) mit dem Rahmen (101) verbunden ist, und wobei mittels dieser Faltungs-Fixierungen (107,108) die erste und die zweite Faltung des Tuches unter Bildung einer Falte (105) erfolgt.

6. Tenniswand (100) nach Anspruch 5, **dadurch gekennzeichnet, dass** die Form der Falte (105) durch eine am Rahmen (101) angebrachte und in ihrer Position variable erste und/oder variable zweite Faltungs-Fixierung (107, 108) einstellbar ist.

7. Tenniswand (100) nach einem der Ansprüche 1 bis 6, **dadurch gekennzeichnet, dass** die Verweilzeit eines Tennisballes in der Vertiefung (105) in einem Bereich von 0,1 bis 3 Sekunden liegt.

8. Verwendung einer Tenniswand nach einem der Ansprüche 1 bis 7 als Hilfsmittel im Tennistraining.

## Claims

1. A tennis wall (100), comprising
- a frame (101) that can be placed on the floor and
- a cloth (102) stretched in this frame (101),
wherein the cloth (102) is stretched such that a flat cloth surface (104) is formed, that an angle α in the range of 25 to 70 degrees is included between the flat cloth surface (104) and the ground, and that a tennis ball hitting the cloth surface (104) rolls or slides up the cloth surface (104) and subsequently rolls or slides down again,
wherein a recess (105) is provided at the upper end (103) of the cloth (102), which recess is formed by a fold (105) in the upper region (106) of the cloth (102) and which has the shape of a trench which runs in the horizontal direction from the left to the right side of the cloth (102) and extends over the entire width of the cloth (102),
**characterized in that** the recess (105) comprises a first cloth portion surface (104B) immediately adjacent the upper end (103) of the planar cloth surface (104), the first cloth portion surface (104B) including an angle β between itself and the ground which is reduced compared to the angle a,
wherein the recess (105) is designed and arranged in such a way that a tennis ball, when it has rolled or slid up the cloth surface (104), subsequently enters the recess (105), rolls or slides out of the recess (105) again along this first cloth part surface (104B), which is less inclined than the flat cloth surface (104), and only then rolls or slides down the cloth surface (104).

2. The tennis wall (100) according to claim 1, **characterized in that** the fold (105) is formed by at least two folds of the cloth (102).

3. The tennis wall (100) according to claim 2, **characterized in that** a first and a second fold form a planar main cloth surface (104A), the first planar cloth part surface (104B) adjacent to the main cloth surface (104A) and a second planar cloth part surface (104C) adjacent to the first planar cloth part surface (104B), whereby
- an angle β is included between the first planar cloth part surface (104B) and the ground which is reduced by at least 5 degrees compared to the angle a;
- an angle γ is included between the second flat cloth part surface (104C) and the floor which is increased by at least 75 degrees compared to the angle a.

4. The tennis wall according to claim 3, **characterized in that** the angle β is in a range of 5 to 30 degrees and the angle γ is in a range of 130 to 190 degrees.

5. The tennis wall (100) according to claim 3 or 4, **characterized in that** the cloth (102) is connected to the frame (101) at the left and right sides of the upper end of the cloth (102) via a first folding fixing (107) and a second folding fixing (108) respectively, and wherein the first and second folding of the cloth is effected by means of these folding fixings (107, 108), forming a fold (105).

6. The tennis wall (100) according to claim 5, **characterized in that** the shape of the fold (105) is adjustable by means of a variable first and/or variable second fold fixation (107, 108) attached to the frame (101) and variable in its position.

7. The tennis wall (100) according to any one of claims 1 to 6, **characterized in that** the dwell time of a tennis ball in the cavity (105) is in a range of 0.1 to 3 seconds.

8. Use of a tennis wall according to any of claims 1 to 7 as an aid in tennis training.

## Revendications

1. Mur de tennis (100), comprenant
- un cadre (101) qui peut être placé sur le sol, et
- un tissu (102) tendu dans ce cadre (101),
dans lequel le tissu (102) est étiré de telle sorte qu'une surface de tissu plate (104) est formée, qu'un angle α dans la plage de 25 à 70 degrés est inclus entre la surface de tissu plate (104) et le sol, et qu'une balle de tennis frappant la surface de tissu (104) roule ou glisse vers le haut de la surface de tissu (104) et roule ou glisse ensuite à nouveau vers le bas,
dans lequel un évidement (105) est prévu à l'extrémité supérieure (103) du tissu (102), lequel évidement est formé par un pli (105) dans la région supérieure (106) du tissu (102) et a la forme d'une tranchée qui s'étend dans la direction horizontale du côté gauche au côté droit du tissu (102) et s'étend sur toute la largeur du tissu (102),
**caractérisé en ce que** l'évidement (105) comprend une première surface de partie de tissu (104B) immédiatement adjacente à l'extrémité supérieure (103) de la surface de tissu plane (104), la première surface de partie de tissu (104B) comprenant un angle β entre elle-même et le sol qui est réduit par rapport à l'angle a,
dans lequel l'évidement (105) est conçu et disposé de telle sorte qu'une balle de tennis, après avoir roulé ou glissé le long de la surface de tissu (104), pénètre ensuite dans l'évidement (105), roule ou glisse à nouveau hors de l'évidement (105) le long de cette première surface de partie de tissu (104B), qui est moins inclinée que la surface de tissu plane (104), et seulement ensuite roule ou glisse le long de la surface de tissu (104).

2. Paroi de tennis (100) selon la revendication 1, **caractérisée en ce que** le pli (105) est formé par au moins deux plis de la toile (102).

3. Mur de tennis (100) selon la revendication 2, **caractérisé en ce qu'**un premier et un second pli forment une surface principale de tissu plane (104A), la première surface de partie de tissu plane (104B) adjacente à la surface principale de tissu (104A), et une seconde surface de partie de tissu plane (104C) adjacente à la première surface de partie de tissu plane (104B), dans lequel
- un angle β est inclus entre la première surface d'élément de tissu plan (104B) et le sol, l'angle β étant réduit de l'angle α d'au moins 5 degrés ;
- un angle γ est inclus entre la seconde surface plane d'élément de tissu (104C) et le sol, l'angle γ étant augmenté de l'angle α d'au moins 75 degrés.

4. Le mur de tennis de la revendication 3, **caractérisé en ce que** l'angle β est compris entre 5 et 30 degrés et l'angle γ est compris entre 130 et 190 degrés.

5. Paroi de tennis (100) selon 3 ou 4, **caractérisée en ce que** la toile (102) est reliée au cadre (101) sur les côtés gauche et droit de l'extrémité supérieure de la toile (102) par l'intermédiaire d'une première fixation de pliage (107) et d'une seconde fixation de pliage (108) respectivement, et dans laquelle, au moyen de ces fixations de pliage (107, 108), le premier et le second pliage de la toile ont lieu avec la formation d'un pli (105).

6. Paroi de tennis (100) selon la revendication 5, **caractérisée en ce que** la forme du pli (105) est ajustable au moyen d'une première et/ou d'une deuxième fixation de pli (107, 108) variable fixée au cadre (101) et variable en position.

7. Mur de tennis (100) de l'une quelconque des revendications 1 à 6, **caractérisé en ce que** le temps de séjour d'une balle de tennis dans la cavité (105) est dans une plage de 0,1 à 3 secondes.

8. utilisation d'un mur de tennis selon l'une quelconque des revendications 1 à 7 comme aide à l'entraînement de tennis.
